# EUROPEAN PATENT SPECIFICATION

(11) **EP 3 818 956 B1**
(45) Date of publication and mention of the grant of the patent: **02.10.2024**
(21) Application number: 20205947.3
(22) Date of filing: 05.11.2020
(51) Int. Cl.: A61B 90/90, A61B 90/96, A61B 90/94, A61B 17/135

(54) **OPTICAL TOURNIQUET INTERFACE FOR SAFE PERSONALIZATION**
OPTISCHE TOURNIQUETSCHNITTSTELLE ZUR SICHEREN PERSONALISIERUNG
INTERFACE DE TOURNIQUET OPTIQUE POUR UNE PERSONNALISATION SÉCURISÉE

(30) Priority: 05.11.2019 US 201962931102 P; 27.02.2020 US 202016803939; 23.07.2020 US 202016937488
(43) Date of publication of application: 12.05.2021
(73) Proprietor: Western Clinical Engineering Ltd, Vancouver BC V6H 1C3 (CA)
(72) Inventor: MCEWEN, James Allen, Vancouver, BC V6H 1C3 (CA); JAMESON, Michael, Vancouver, BC V6H 1C3 (CA); LAU, Tom Yu Chia, Vancouver, BC V6H 1C3 (CA); LIM Rebecca Nicole, Vancouver, BC V6H 1C3 (CA); PROKOPICH Nicholas Alexander, Vancouver, BC V6H 1C3 (CA)
(74) Representative: Forresters IP LLP

(56) References cited:
- US-A1- 2009 118 628
- US-A1- 2013 218 033
- US-A1- 2015 272 452
- US-A1- 2019 206 562
- US-B1- 9 039 730
- US-B2- 6 682 547

## Description

### CROSS REFERENCE TO RELATED APPLICATION

This application claims priority from U.S. Patent Application No. 16/803,939, filed February 27, 2020, which claims the benefit of U.S. Provisional Patent Application No. 62/931,102, filed November 5, 2019, and also U.S. Patent application no. 16/937,488 filed on July 23, 2020.

### BACKGROUND

Surgical tourniquet systems are commonly used to stop the flow of arterial blood into a portion of a patient's limb, thus creating a clear, dry surgical field that facilitates the performance of a surgical procedure and improves outcomes. A typical surgical tourniquet system of the prior art includes a tourniquet cuff for encircling a patient's limb at a desired location, a tourniquet instrument, and flexible tubing connecting the cuff to the instrument. In some surgical tourniquet systems of the prior art, the tourniquet cuff includes inflatable bladder that is connected pneumatically to a tourniquet instrument via flexible tubing attached to one or two cuff ports. The tourniquet instrument includes a pressure controller operable for automatically controlling pressure near a reference pressure in the connected inflatable bladder during a pressure control time period. Many types of such pneumatic surgical tourniquet systems have been described in the prior art, such as those described by McEwen in U.S. Pat. No. 4,469,099, U.S. Pat. No. 4,479,494, U.S. Pat. No. 5,439,477 and by McEwen and Jameson in U.S. Pat. No. 5,556,415 and U.S. Pat. No. 5,855,589.

Many studies published in the medical literature have shown that the safest tourniquet pressure is the lowest pressure that will stop the penetration of arterial blood past a specific cuff applied to a specific patient for the duration of that patient's surgery. Such studies have shown that higher tourniquet pressures are associated with higher risks of tourniquet-related injuries to the patient. Therefore, when a tourniquet is used in surgery, surgical staff generally try to use the lowest tourniquet pressure that in their judgment is safely possible.

It is well established in the medical literature that the optimal guideline for setting the pressure of a constant-pressure tourniquet is based on "Limb Occlusion Pressure" (LOP). LOP can be defined as the minimum pressure required, at a specific time in a specific tourniquet cuff applied to a specific patient's limb at a specific location, to stop the flow of arterial blood into the limb distal to the cuff. LOP is affected by variables including the patient's limb characteristics, characteristics of the selected tourniquet cuff, the technique of application of the cuff to the limb, physiologic characteristics of the patient including blood pressure and limb temperature, and other clinical factors (for example, the extent of any elevation of the limb during LOP measurement and the extent of any limb movement during LOP measurement).

The currently established guideline for setting tourniquet pressure based on LOP is that an additional safety margin of pressure is added to the measured LOP, in an effort to account for variations in physiologic characteristics and other changes that may be anticipated to occur normally over the duration of a surgical procedure.

LOP can be measured automatically using a distal flow sensor as described by McEwen in U.S. Pat. No. 7,479,154, or automatically using a dual-purpose cuff, as described by McEwen in U.S. Pat. No. 9,931,126.

Automatic measurement of LOP using a distal sensor, as described by McEwen in U.S. Pat. No. 7,479,154, utilizes a blood flow transducer that employs a photoplethysmographic principle to sense blood flow in the limb distal to the applied tourniquet cuff.

Automatic LOP measurement using a dual-purpose cuff has many advantages over the distal sensor method, as described in McEwen in U.S. Pat. No. 9,931,126. However, to obtain accurate and reliable LOP measurements, this method requires the use of a validated tourniquet cuff, suitable as both a sensor and an effector (dual-purpose). If a non-validated tourniquet cuff (not dual-purpose) is used, then the parameters for the LOP measurement may not be suitable, causing inaccurate LOP values that may result in bleed through or excessively high pressures applied, causing significant risk to the patient.

In addition, characteristics of the tourniquet cuff, such as cuff width and cuff length, whether the cuff is dual-port or dual-bladder, can be used to optimize the parameters for both the LOP measurement using a distal sensor or a dual-purpose cuff as described by McEwen in U.S. Pat. No. 7,479,154 and McEwen in U.S. Pat. No. 9,931,126, respectively; adjust the LOP safety margin; and change tourniquet settings, such as reducing the maximum reference pressure if the tourniquet cuff is a pediatric cuff.

Whether a cuff is a dual-purpose cuff or not, and the characteristics of a tourniquet cuff are examples of personalization parameters that are cuff-related that can be used to personalize and optimally configure the tourniquet instrument to increase patient safety. These personalization parameters may be entered manually into the instrument, which can be time consuming. Alternatively, as described in this invention, they may be read by the instrument automatically through an optical tourniquet interface.

Other personalization parameters that are not cuff-related may also be used. For instance, personalization parameters included in safety protocols can also be used to optimally configure the tourniquet instrument to increase patient safety by personalizing the tourniquet settings to the patient, the surgical procedure, or the surgeon. These personalization parameters as part of a safety protocol may include pressure and time settings, whether LOP measurement is required, LOP safety margin values, and maximum reference pressure. A user may select a safety protocol suitable for the patient, the surgical procedure, or the surgeon from a list of safety protocols to automatically configure the tourniquet settings.

In U.S. Pat. No. 9,931,126, McEwen et al. describe a surgical tourniquet system with a single channel for a single cuff. However, tourniquet systems are also commonly used with two channels for two cuffs or for a single cuff with two bladders. These multi-cuff or multi-bladder tourniquet systems are commonly used for surgeries involving intravenous regional anesthesia (IVRA) or bilateral procedures. In IVRA procedures, a dual-bladder cuff or a two-cuff system is used to retain an anesthetic agent after its introduction within a desired area. If the reference pressure levels or the inflation and deflation times of the dual-bladder cuff or a two-cuff system are not set properly, the anesthetic agent may enter the patient's circulatory system, causing serious injury or death. Tourniquet settings specifying the safe inflation and deflation times of the dual-bladder cuff or a two-cuff system can be specified in safety protocols.

In some surgical procedures, it is desirable to follow specific deflation sequences personalized to the patient, the surgical procedure or the surgeon. When a tourniquet cuff has been applied on a limb for a long duration, perioperative staff may deflate the tourniquet cuff for a short time period then re-inflate to allow limb reperfusion. A surgeon may desire a gradual stepped decrease in cuff pressure to control the release of toxins and metabolites. A procedure may also require the temporary reduction of pressure to check for bleeding at the surgical site. These deflation sequences can also be specified in safety protocols as another personalization parameter.

Safety protocols may be entered manually into the instrument, which can be time consuming. Alternatively, as described by this invention, safety protocol may be created through a remote device such as through an app on a mobile device then read by the instrument automatically through an optical tourniquet interface.

Since different tourniquet instruments may use different methods of determining LOP, and regulate pressure, due to hardware and/or software differences, personalization parameters described previously may be suitable for optimally configuring one type of tourniquet instrument but unsuitable or hazardous for another type of tourniquet instrument. For an example, personalization parameters intended for a single-port tourniquet instrument would not be suitable for a dual-port tourniquet instrument. Furthermore, configuring a tourniquet instrument by reading personalization parameters, such as those contained in a safety protocol, may be hazardous in certain situations, such as when the cuff is pressurized during a surgical procedure.

Therefore, there is a need for an apparatus and method to acquire personalization parameters to optimally configure a tourniquet instrument to increase patient safety by personalizing the tourniquet settings to the patient, the surgical procedure, or the surgeon, only if the personalization parameters are intended for the tourniquet instrument.

Some surgical tourniquet systems of the prior art include means for configuring the tourniquet instrument through cuff identification. McEwen in U.S. Pat. No. 6,682,547 describes a cuff identification method in which the tourniquet instrument detects cuff connectors having different colors that are indicative of the physical characteristics of the cuff, after the cuff and the instrument establishes pneumatic connection. This method has several limitations: (1) the color detection is performed at the cuff connector which is away from the instrument. Thus, the hardware used for detection is carried on the pneumatic tubing connected to the instrument and is more susceptible to damage as the pneumatic tubing may be dropped onto the floor, stepped on, or come in contact with liquids such as blood and cleaning solutions; (2) the number of detectable colored connectors are limited by their distances from each other in color space. The greater the number of different colored connectors there are, the closer they are to each other in color space, and the more likely it would be for false positives under different lighting conditions, or as the color degrades over time or due to cleaning chemicals. Therefore, the number of different cuffs that can be identified and the amount of data that can be contained in the colored connectors are limited; and (3) cuff detection requires physical contact between the cuff and the instrument through the connectors which may be hazardous if the cuff is sterile and must remain sterile.

Contactless cuff identification through RFID is described by McEwen in U.S. Pat. No. 9,931,126. However, RFID cuff identification has limitations: (1) RFID tags are susceptible to malfunction upon irradiation commonly used to sterilize tourniquet cuff assemblies; (2) misidentification may occur when more than one cuff with a different RFID tag is in close proximity to the RFID reader; and (3) RFID reader and RFID tags may incur a substantial increase in the cost of the instrument and/or the tourniquet cuff.
US2015/272452 discloses a system configured to determine a characteristic of a patient, the system including a plurality of sensors, wherein each sensor of the plurality of sensors is configured to noninvasively determine a respective parameter of the patient, and wherein the parameter determined by each sensor is different from parameters determined by remaining sensors of the plurality of sensors. The disclosed system also includes a connector having a passage configured to direct pressurised fluid therethrough, wherein the plurality of sensors is connected to the connector.
US9039730 discloses a method and apparatus for regulating tourniquet cuff pressure based on a personalized tourniquet pressure (PTP) to facilitate safe performance of a surgical procedure. The apparatus includes a dual-purpose tourniquet cuff adapted to encircle a region of a patient limb to provide both a sensor during a pre-surgical time period and an effector during a surgical time period. An effector module communicates pneumatically with an inflatable bladder of the cuff for maintaining pressure in the bladder near a PTP during the surgical time period to safely stop penetration of arterial blood past the cuff. A pulsation sensor that communicates pneumatically with the inflatable bladder during the pre-surgical time period senses and characterizes pressure pulsations that are indicative of penetration of arterial blood into the region of the limb encircled by the cuff. A PTP estimator is responsive to the pulsation sensor for producing an estimate of the PTP, such that the estimate of the PTP is a function of the sensed pulsations.
US2019/206562 discloses a method of displaying an operational parameter of a surgical system is disclosed. The method includes receiving, by a cloud computing system of the surgical system, first usage data, from a first subset of surgical hubs of the surgical system; receiving, by the cloud computing system, second usage data, from a second subset of surgical hubs of the surgical system; analyzing, by the cloud computing system, the first and the second usage data to correlate the first and the second usage data with surgical outcome data; determining, by the cloud computing system, based on the correlation, a recommended medical resource usage configuration; and displaying, on respective displays on the first and the second subset of surgical hubs, indications of the recommended medical resource usage configuration.

Other cuff identification apparatus and methods described or suggested in the prior art may have high risk of malfunction, be unreliable, add substantial cost, have limited number of cuffs that can be identified, and/or create legacy issues.

### SUMMARY

Described below are implementations of an optical tourniquet interface for safe personalization that address shortcomings of conventional approaches.

According to a first implementation, a tourniquet system according to claim 1 is provided.

### BRIEF DESCRIPTION OF THE DRAWINGS

FIG. 1 is a block diagram of one implementation of a tourniquet system having an optical interface for safe personalization.
FIG. 2A is a schematic plan view showing a sterile tourniquet cuff assembly having a machine-readable instrument symbol, machine-readable personalization data and a sterile pediatric cuff.
FIG. 2B is a pictorial representation of the sterile tourniquet cuff assembly from FIG. 2A being used with an instrument.
FIG. 3A is a drawing of a representative remote device having a machine-readable remote device symbol and a machine-readable data.
FIG. 3B is a pictorial representation of the remote device from FIG. 3A being used with the instrument.
FIG. 4 is a pictorial representation of a remote device displaying a clinical summary of a surgery for future analysis as received from the instrument.

### DETAILED DESCRIPTION

Described below are implementations of a tourniquet system that can acquire personalization parameters for safe tourniquet personalization, such as only if the personalization parameters are intended for the tourniquet instrument. Such a system includes an optical tourniquet interface that can contactlessly read a machine-readable instrument symbol that identifies one type of tourniquet instrument from a plurality of types of tourniquet instruments, and a machine-readable personalization data representing one or more values of personalization parameters on a tourniquet cuff package or a remote device. Such a system presents in a form perceptible to a user the value(s) of the personalization parameter(s) if the machine-readable instrument symbol matches stored authentication data. Such a system includes a safe transfer key, operable by the user to manually accept and transfer the value(s) of the personalization parameter(s) to the pressure controller. Such a system may also include a reject key or other control element, operable by the user to reject and prevent the transfer of the value(s) of the personalization parameter(s) to a pressure controller. Such a system may automatically initiate actions, such as inflation or deflation, immediately or shortly after the manual or automatic transfer of the value(s) of the personalization parameter(s) to the pressure controller

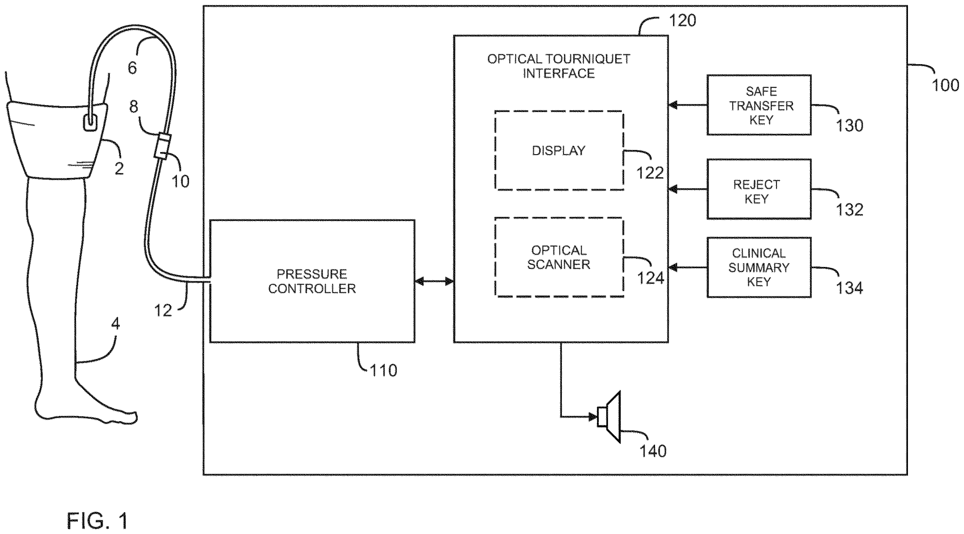

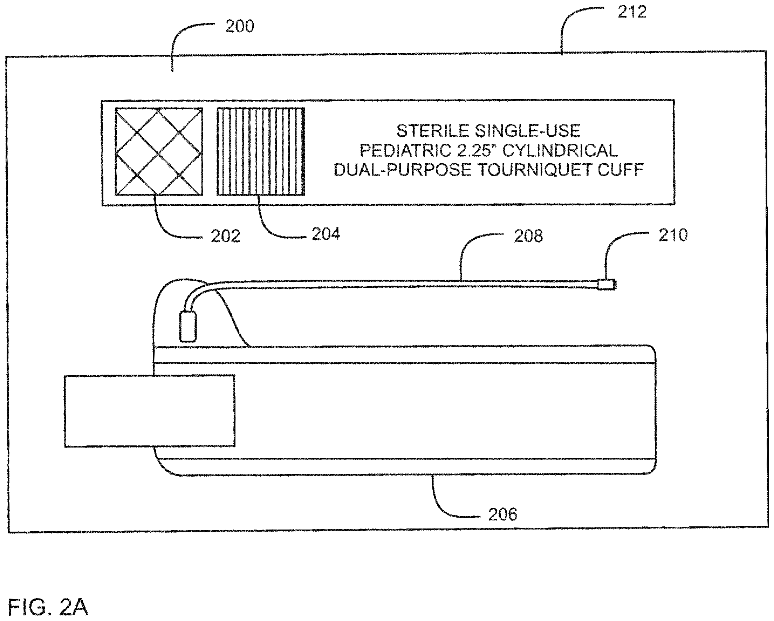

FIG. 1 depicts a block diagram of the tourniquet system of one representative implementation. Cuff 2 having inflatable bladder is shown encircling a limb 4 of patient. Cuff 2 may be supplied to a user as a single-use sterile cuff, packaged in a sterile tourniquet cuff assembly (see FIG. 2A), or it may be a reusable cuff. A pneumatic passageway between instrument 100 and cuff 2 is provided by cuff port 6, male locking connector 8, female locking connector 10 and flexible tubing 12. Cuff port 6 is fitted with a male locking connector 8 that mates to form a releasable pneumatic connection with female locking connector 10. Instrument 100 comprises of pressure controller 110, optical tourniquet interface 120, safe transfer key 130, reject key 132, clinical summary key 134, and speaker 140.

Pressure controller 110 includes a pneumatic pump and valve assembly and is operable for automatically controlling pressure in the connected inflatable bladder of cuff 2 near a reference pressure during a pressure control time period suitably long for a surgical procedure. Pressure controller 110 is adapted to automatically measure Limb Occlusion Pressure (LOP) of limb 4 with cuff 2.

Pressure controller 110 is also adapted to respond to values of personalization parameters transferred from optical tourniquet interface 120 to improve the safety and effectiveness of the pressure regulation, automatic LOP measurement, and other tourniquet settings. Personalization parameters include: whether cuff 2 is a dual-purpose cuff suitable for automatic LOP measurement or not; whether cuff 2 is adapted for use in a surgical procedure involving intravenous regional anesthesia or not; whether cuff 2 is single-use or reusable; the size and shape of the limb for which cuff 2 is intended to be applied to; pressure and time settings; whether LOP measurement is required; LOP safety margin values; maximum reference pressure; and other.

Pressure controller 110 records cuff 2's pressure, time, and alarm history during a surgical procedure as a plurality of pressures and alarm events of cuff 2 for a plurality of times during the pressure control time period. Upon completion of the surgical procedure, i.e., at the end of the pressure control time period, and upon activation of clinical summary key 134, optical tourniquet interface 120 communicates with pressure controller 110 to display the pressure, time, and alarm history of cuff 2 on display 122. To enable a user to capture this information remotely for later analysis, optical tourniquet interface 120 may also encode and display the information in a machine-readable form that can be optically read by a remote device, as described below. Optical tourniquet interface 120 may also encode and display values of personalization parameters in a machine-readable form.

Optical tourniquet interface includes display 122, and optical scanner 124. Display 122 may include a touchscreen to allow a user to interface with optical tourniquet interface 120. Optical tourniquet interface 120 communicates with pressure controller 110 to allow a user to control the operation of instrument 100.

Display 122 displays information to the user including reference pressure, current pressure, elapsed time, and alarm messages. Optical scanner 124 is adapted to contactlessly acquire values of personalization parameters for safe personalization, only if the personalization parameters are intended for instrument 100, as described below. If the personalization parameters are not intended for instrument 100, pressure controller 110 may use a predetermined stored value of the personalization parameter. In addition, optical tourniquet interface 120 may notify to the user through display 122 and/or speaker 140.

Once values of personalization parameters are acquired, instrument 100 allows the user to activate safe transfer key 130 to selectively transfer the values of the personalization parameters to pressure controller 110. Instrument 100 further allows the user to activate reject key 132 to reject transfer of the values of personalization parameters to pressure controller 110.

Safe transfer key 130, reject key 132, and clinical summary key 134 may be mechanical buttons on instrument 100 or they may be incorporated as touchable keys on a touchscreen of display 122.

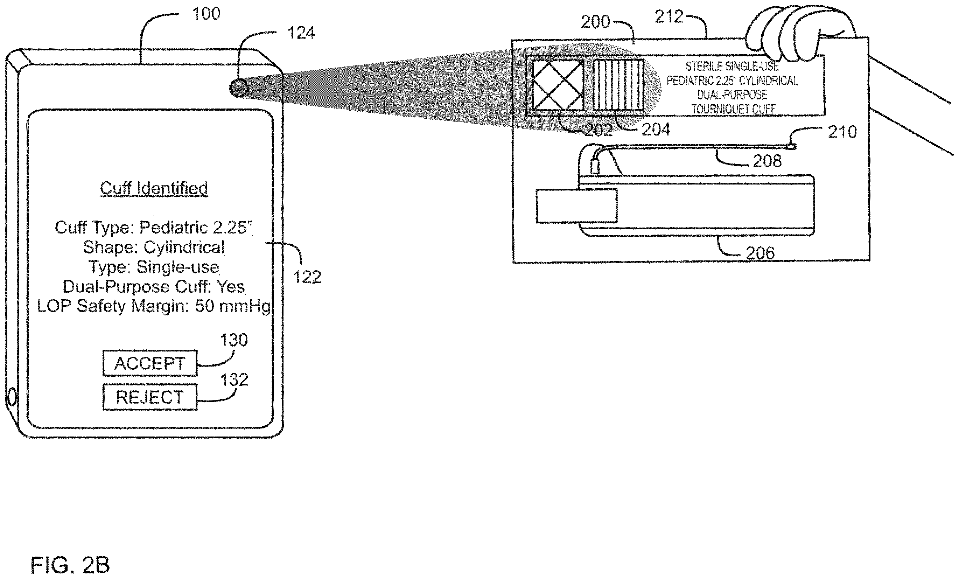

FIGS. 2A and 2B depict an example of the preferred embodiment in use.

FIG. 2A shows a sterile tourniquet cuff assembly 200 having machine-readable instrument symbol 202, machine-readable personalization data 204, and sterile single-use pediatric cuff 206 having an inflatable bladder adapted for connection to instrument 100. Cuff 206 includes sterile cuff port 208 and sterile male locking connector 210. Sterile male locking connector 210 is adapted for releasably connecting cuff 206 to instrument 100. Sterile tourniquet cuff assembly 200 includes a sterile barrier 212 to ensure the sterility of cuff 206 before use.

Machine-readable instrument symbol 202 and machine-readable personalization data 204 may be texts, images, barcodes or other marks identifiable and readable by optical scanner 124. Machine-readable instrument symbol 202 and machine-readable personalization data 204 may be encoded together in a single marking instead of two separate markings. Different techniques of encoding will be apparent to those skilled in the art. Machine-readable instrument symbol 202 and machine-readable personalization data 204 may be located on a tourniquet cuff assembly, as shown in FIG. 2A, and/or on a cuff itself, for instance, as part of a label on a reusable cuff.

Machine-readable personalization data 204 represents at least one value of a personalization parameter that is used to optimally configure instrument 100 to increase patient safety by personalizing the tourniquet settings for an individual patient, surgical procedure, or surgeon. In this example, personalization parameters include information indicating cuff 206 is a single-use, cylindrical pediatric cuff with a cuff width of 2.25". Furthermore, cuff 206 is a dual-purpose cuff and thus automatic LOP measurement using a dual-purpose cuff is enabled. Since the detected cuff is a pediatric cuff, the LOP safety margin is 50 mmHg.

Since different tourniquet instruments may use different methods of determining LOP, and regulate pressure, due to hardware and/or software differences, personalization parameters may be suitable for optimally configuring one type of tourniquet instrument but unsuitable or hazardous for another type of tourniquet instrument. For an example, personalization parameters intended for a single-port tourniquet instrument would not be suitable for a dual-port tourniquet instrument. Machine-readable instrument symbol 202 identifies the type of tourniquet instrument that is suitable for using the values of personalization parameters represented by machine-readable personalization data 204 from a plurality of tourniquet instruments.

FIG. 2B shows instrument 100 with display 122 and optical scanner 124. When a user positions sterile tourniquet cuff assembly 200 within the field of view of optical scanner 124, optical scanner contactlessly reads machine-readable instrument symbol 202 and machine-readable personalization data 204. The field of view of optical scanner 124 is optimized to encompass a predetermined range of distances and a predetermined range of orientations relative to optical scanner 124 to prevent inadvertent hazardous reading of a second tourniquet cuff assembly outside the predetermined ranges.

Optical tourniquet interface 120 authenticates machine-readable instrument symbol 202 by matching it to stored authentication data. Once machine-readable instrument symbol 202 is authenticated, optical tourniquet interface 120 displays the values of the personalization parameters through display 122. The user may review the values of the personalization parameters and if they are considered safe and appropriate for the surgical procedure, the user can then selectively transfer the presented values of the personalization parameters to pressure controller 110 through safe transfer key 130. The user may also reject the transfer of the presented values of the personalization parameters to pressure controller 110 through reject key 132.

If machine-readable instrument symbol 202 does not match stored authentication data, pressure controller 110 may use predetermined stored values of personalization parameters, or prompt the user to manually enter values of personalization parameters. In addition, optical tourniquet interface 120 may indicate to the user that an unknown machine-readable instrument symbol has been read through display 122 and/or speaker 140.

To ensure tourniquet settings are not altered inadvertently in certain situations which may be hazardous, such as changing the reference pressure while cuff 206 is pressurized, safe transfer key 130 only allows the transfer of the values of the personalization parameters to pressure controller 110 when pressure controller 110 is inoperable. Optical tourniquet interface 120 may also prevent optical scanner 124 from scanning machine-readable instrument symbol 202 and machine-readable personalization data 204 when pressure controller 110 is operable.

Once the values of the personalization parameters are transferred to pressure controller 110, instrument 100 may immediately utilize the values of the personalization parameters and initiate certain actions, such as inflation or a measurement of LOP. Alternatively, instrument 100 may wait for additional user inputs before initiating new actions.

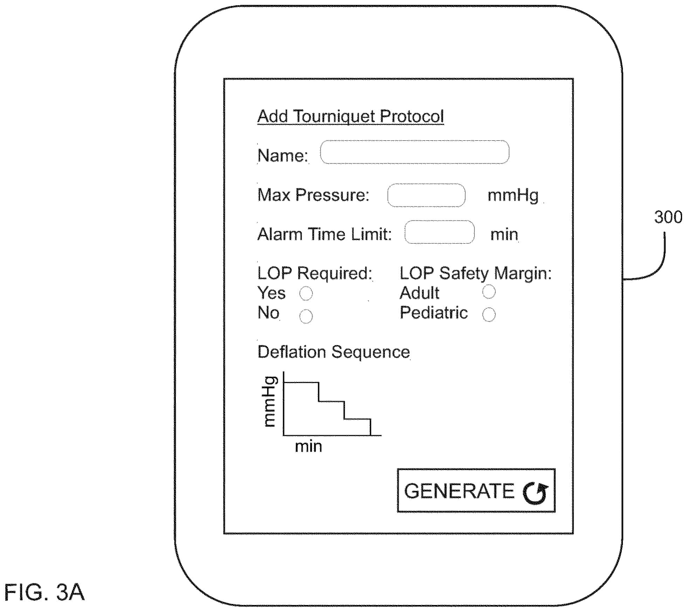

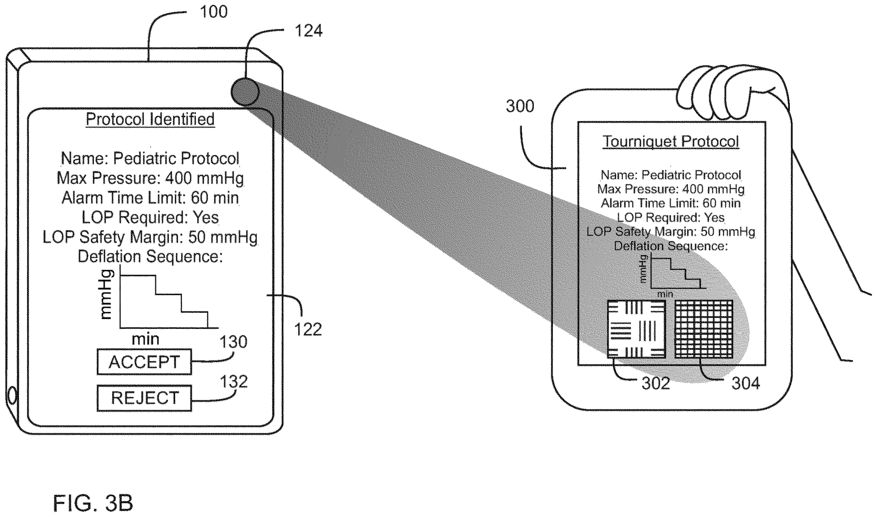

FIGS. 3A and 3B depict another example of the preferred embodiment in use.

FIG. 3A shows remote device 300 having a touchscreen for a user to generate a safety protocol. Safety protocol includes personalization parameters that can be used to optimally configure instrument 100 to increase patient safety by personalizing the tourniquet settings to the patient, the surgical procedure, or the surgeon. FIG. 3 A shows remote device 300 allowing the user to define the following personalization parameters: the name of the protocol; the maximum reference pressure limit; the alarm time limit; whether LOP measurement is required; whether LOP safety margin is for an adult or a pediatric patient; and the deflation sequence. In the preferred embodiment, remote device 300 is a smart-phone.

FIG. 3B shows instrument 100 with display 122 and optical scanner 124. After the generation of a safety protocol from FIG. 3A, remote device 300 shows machine-readable remote device symbol 302 corresponding to remote device 300, and machine-readable data 304 associated with remote device 300 that is indicative of at least one remote value of a personalization parameter of the developed safety protocol.

When a user positions remote device 300 within the field of view of optical scanner 124, optical scanner contactlessly reads machine-readable remote device symbol 302 and machine-readable data 304. The field of view of optical scanner 124 is optimized to encompass a predetermined range of distances and a predetermined range of orientations relative to optical scanner 124 to prevent inadvertent hazardous reading of a second remote device outside the predetermined ranges.

Optical tourniquet interface 120 authenticates machine-readable remote device symbol 302 by matching it to stored authentication data. Once machine-readable remote device symbol 302 is authenticated, optical tourniquet interface 120 displays the values of the personalization parameters through display 122. The user may review the values of the personalization parameters and if they are considered safe and appropriate for the surgical procedure, the user can then selectively transfer the presented values of the personalization parameters to pressure controller 110 through safe transfer key 130. The user may also reject the transfer of the presented values of the personalization parameters to pressure controller 110 through reject key 132. In this example, personalization parameters include information associated with a safety protocol named "pediatric protocol" such as the maximum reference pressure limit (400 mmHg), alarm time limit (60 min), whether LOP is required or not (yes), to use pediatric LOP safety margin, and to use a stepped-decrease deflation sequence, which may be used to facilitate the detection and closure of bleeding vessels.

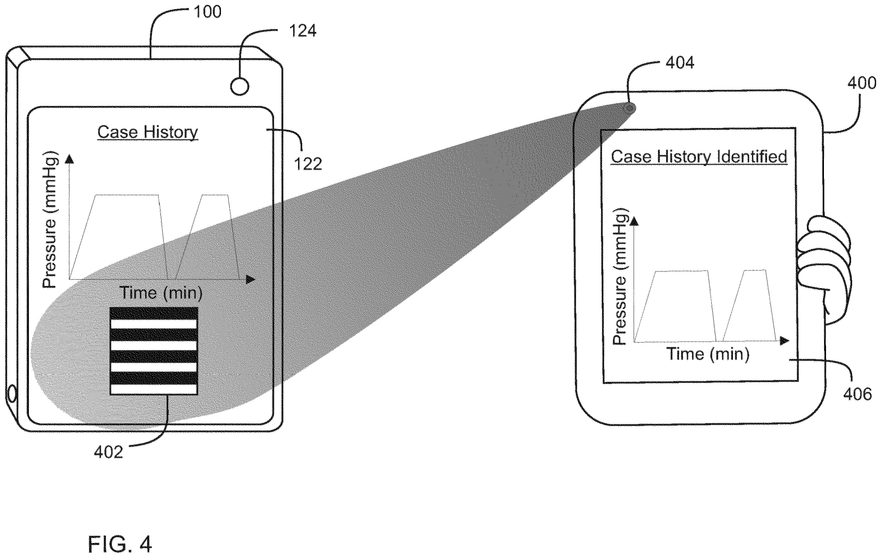

FIG. 4 depicts another example of the preferred embodiment in use.

FIG. 4 shows instrument 100 after a user activated clinical summary key 134. Clinical summary key 134 may be activated by the user after a pressure control time period, such as one suitably long for a surgical procedure. Display 122 displays a graphical representation of plurality of pressure levels and alarm events corresponding to a plurality of times during the pressure control time period. Instrument 100 also displays machine-readable clinical data 402 that is indicative of the graphical representation. Machine-readable clinical data 402 may also be indicative of previously transferred values of personalization parameters. Remote personalization device 400 is shown with remote scanner 404 capable of reading machine-readable clinical data 402, and displaying the information contained in machine-readable clinical data 402 on remote display 406. In the preferred embodiment, remote device 400 is a smart phone.

The embodiments illustrated are not intended to be exhaustive or to limit the invention to the precise form disclosed. They are chosen and described in order to explain the principles of the invention and its application and practical use, and thereby enable others skilled in the art to utilize the invention.

In view of the many possible embodiments to which the disclosed principles may be applied, it should be recognized that the illustrated embodiments are only preferred examples and should not be taken as limiting in scope. Rather, the scope of protection is defined by the following claims.

## Claims

1. A tourniquet system having an optical tourniquet interface (120) for safe personalization, comprising:
a tourniquet cuff assembly (200) including:
a tourniquet cuff (2, 206) having an inflatable bladder adapted for connection to a tourniquet instrument,
a contactlessly-readable instrument symbol (202) identifying one type of tourniquet instrument from a plurality of types of tourniquet instruments, and
contactlessly-readable personalization data (204) representing a value of a personalization parameter for safe operation of the tourniquet cuff (2, 206) when connected to the identified type of tourniquet instrument; and
a tourniquet instrument including:
an optical tourniquet interface (120) communicating with a pressure controller (110) and operable for contactlessly reading and authenticating the contactlessly-readable instrument symbol (202) associated with the tourniquet cuff (2, 206) if it matches stored instrument authentication data, and further operable for contactlessly reading the contactlessly-readable personalization data (204) associated with the cuff (2, 206),
wherein the optical tourniquet interface (120) is adapted to present in a form perceptible to a user the value of the personalization parameter if the contactlessly-readable instrument symbol (202) has been authenticated;
a safe transfer key (130) which is operable by the user to manually selectively transfer the presented value of the personalization parameter to the pressure controller (110) only if the pressure controller (110) is inoperable; and
the pressure controller (110) releasably connectable to the tourniquet cuff (2, 206) and responsive to the transferred value of the personalization parameter, wherein the pressure controller (110) is operable for automatically controlling a level of pressure in the connected tourniquet cuff (2, 206) during a pressure control time period.

2. The tourniquet system of claim 1 wherein the tourniquet cuff (2, 206) is sterile, the system further comprising;
a sterile connector (210) adapted for releasably connecting the sterile tourniquet cuff (2, 206) to the identified type of tourniquet instrument; and
wherein the instrument symbol (202) and the personalization data (204) are carried at a location enabling contactless reading of the instrument symbol (202) and the personalization data (204) only if the sterile cuff assembly (200) is positioned by the user within a predetermined range of distances and within a predetermined range of orientations relative to the optical tourniquet interface (120).

3. The tourniquet system of claim 1 or 2 wherein the optical tourniquet interface (120) includes a display (122) and wherein the optical tourniquet interface (120) is adapted to present the value of the personalization parameter to the user by displaying the value on the display (122).

4. The tourniquet system of any preceding claim further including a reject key (132) which is operable by the user to manually selectively reject transfer of the presented value of the personalization parameter.

5. The tourniquet system of claim 2 wherein the value of the personalization parameter indicates that the tourniquet cuff (2, 206) is adapted for automatic measurement of a Limb Occlusion Pressure of a patient by the identified tourniquet instrument.

6. The tourniquet system of claim 2 or 5 wherein the value of the personalization parameter indicates that the tourniquet cuff (2, 206) is adapted for a pediatric patient.

7. The tourniquet system of claim 2, 5 or 6, wherein the value of the personalization parameter indicates that the tourniquet cuff (2, 206) is adapted for intravenous regional anesthesia of a patient's limb.

8. The tourniquet system of any preceding claim wherein the optical tourniquet interface (120) is further operable upon actuation of a clinical summary key by the user after the pressure control time period for generating and displaying contactlessly-readable clinical data indicative of at least one level of pressure corresponding to a time within the pressure control time period, and preferably wherein the contactlessly-readable clinical data is further indicative of the transferred presented value of the personalization parameter.

9. The tourniquet system of any preceding claim further including a remote device (300, 400):
wherein the optical tourniquet interface (120) is further operable for contactlessly reading and authenticating a contactlessly-readable remote device symbol (302) associated with the remote device (300, 400) if it matches stored authentication data corresponding to the remote device (300, 400); and
wherein the optical tourniquet interface (120) is additionally operable for contactlessly reading contactlessly-readable data associated with the remote device (300, 400) that is indicative of a remote value of a personalization parameter;
wherein the safe transfer key (130) is further adapted for enabling the user to safely transfer the remote value of the personalization parameter to the pressure controller (110) only if the contactlessly-readable remote device symbol (302) has been authenticated and only when the pressure controller (110) is inoperable; and
wherein the pressure controller (110) is further responsive to the transferred remote value for safely controlling pressure in the connected tourniquet cuff during the pressure control time period.

10. The tourniquet system of any preceding claim, wherein if the machine-readable instrument symbol (202) is not authenticated, the tourniquet instrument is configured to use a predetermined stored value for at least one personalization parameter.

11. The tourniquet system of any preceding claim wherein the safe transfer key (130) further initiates operation of the pressure controller (110) upon the selective transfer of the presented value of the personalization parameter.

## Patentansprüche

1. Tourniquet-System, das eine optische Tourniquet-Schnittstelle (120) für eine sichere Personalisierung aufweist, umfassend:
eine Tourniquet-Manschettenanordnung (200), die einschließt:
eine Tourniquet-Manschette (2, 206), die eine aufblasbare Blase aufweist, die für eine Verbindung an ein Tourniquet-Instrument angepasst ist,
ein kontaktlos lesbares Instrumentensymbol (202), das eine Art eines Tourniquet-Instruments aus einer Vielzahl von Arten von Tourniquet-Instrumenten identifiziert, und
kontaktlos lesbare Personalisierungsdaten (204), die einen Wert eines Personalisierungsparameters für einen sicheren Betrieb der Tourniquet-Manschette (2, 206) repräsentieren, wenn sie mit der identifizierten Tourniquet-Instrumentenart verbunden werden; und
ein Tourniquet-Instrument, das einschließt:
eine optische Tourniquet-Schnittstelle (120), die mit einem Druckregler (110) kommuniziert und zum kontaktlosen Lesen und Authentifizieren des kontaktlos lesbaren Instrumentensymbols (202) betriebsfähig ist, das mit der Tourniquet-Manschette (2, 206) verknüpft ist, falls es mit gespeicherten Instrumentenauthentifizierungsdaten übereinstimmt, und die ferner zum kontaktlosen Lesen der kontaktlos lesbaren Personalisierungsdaten (204) betriebsfähig ist, die mit der Manschette (2, 206) verbunden sind,
wobei die optische Tourniquet-Schnittstelle (120) angepasst ist, um den Wert des Personalisierungsparameters in einer Form darzustellen, die für einen Benutzer wahrnehmbar ist, falls das kontaktlos lesbare Instrumentensymbol (202) authentifiziert wurde;
einen Schlüssel (130) für eine sichere Übertragung, die durch den Benutzer betriebsfähig ist, um den dargestellten Wert des Personalisierungsparameters nur dann an den Druckregler (110) manuell selektiv zu übertragen, falls der Druckregler (110) nicht betriebsfähig ist; und
wobei der Druckregler (110) mit der Tourniquet-Manschette (2, 206) lösbar verbindbar ist und auf den übertragenen Wert des Personalisierungsparameters reagiert, wobei der Druckregler (110) zum automatischen Steuern eines Drucklevels in der verbundenen Tourniquet-Manschette (2, 206) während eines Druckregelzeitraums betriebsfähig ist.

2. Tourniquet-System nach Anspruch 1, wobei die Tourniquet-Manschette (2, 206) steril ist, das System ferner umfassend;
einen sterilen Verbinder (210), der zum lösbaren Verbinden der sterilen Tourniquet-Manschette (2, 206) mit der identifizierten Art von Tourniquet-Instrument angepasst ist; und
wobei das Instrumentensymbol (202) und die Personalisierungsdaten (204) an einer Stelle getragen werden, die ein kontaktloses Lesen des Instrumentensymbols (202) und der Personalisierungsdaten (204) nur dann ermöglicht, falls die sterile Manschettenanordnung (200) durch den Benutzer innerhalb eines zuvor bestimmten Bereichs von Entfernungen und innerhalb eines zuvor bestimmten Bereichs von Ausrichtungen relativ zu der optischen Tourniquet-Schnittstelle (120) positioniert wird.

3. Tourniquet-System nach Anspruch 1 oder 2, wobei die optische Tourniquet-Schnittstelle (120) eine Anzeige (122) einschließt und wobei die optische Tourniquet-Schnittstelle (120) angepasst ist, um dem Benutzer den Wert des Personalisierungsparameters durch Anzeigen des Werts auf der Anzeige (122) darzustellen.

4. Tourniquet-System nach einem der vorstehenden Ansprüche, das ferner einen Ablehnungsschlüssel (132) einschließt, der durch den Benutzer betriebsfähig ist, um eine Übertragung des dargestellten Werts des Personalisierungsparameters manuell selektiv abzulehnen.

5. Tourniquet-System nach Anspruch 2, wobei der Wert des Personalisierungsparameters angibt, dass die Tourniquet-Manschette (2, 206) für eine automatische Messung eines Extremitätenokklusionsdrucks eines Patienten durch das identifizierte Tourniquet-Instrument angepasst ist.

6. Tourniquet-System nach Anspruch 2 oder 5, wobei der Wert des Personalisierungsparameters angibt, dass die Tourniquet-Manschette (2, 206) für einen pädiatrischen Patienten angepasst ist.

7. Tourniquet-System nach Anspruch 2, 5 oder 6, wobei der Wert des Personalisierungsparameters angibt, dass die Tourniquet-Manschette (2, 206) für eine intravenöse Regionalanästhesie einer Extremität eines Patienten angepasst ist.

8. Tourniquet-System nach einem der vorstehenden Ansprüche, wobei die optische Tourniquet-Schnittstelle (120) ferner bei einer Betätigung eines klinischen Zusammenfassungsschlüssels durch den Benutzer nach dem Druckkontrollzeitraum zum Erzeugen und Anzeigen von kontaktlos lesbaren klinischen Daten betriebsfähig ist, die mindestens einen Druckpegel angeben, der einer Zeit innerhalb des Druckkontrollzeitraums entspricht, und vorzugsweise wobei die kontaktlos lesbaren klinischen Daten ferner den übertragenen dargestellten Wert des Personalisierungsparameters angeben.

9. Tourniquet-System nach einem der vorstehenden Ansprüche, das ferner eine Remote-Vorrichtung (300, 400) einschließt:
wobei die optische Tourniquet-Schnittstelle (120) ferner zum kontaktlosen Lesen und Authentifizieren eines kontaktlos lesbaren Symbols (302) der Remote-Vorrichtung betriebsfähig ist, das mit der Remote-Vorrichtung (300, 400) verknüpft ist, falls es mit gespeicherten Authentifizierungsdaten übereinstimmt, die der Remote-Vorrichtung (300, 400) entsprechen; und
wobei die optische Tourniquet-Schnittstelle (120) zusätzlich zum kontaktlosen Lesen kontaktlos lesbarer Daten, die mit der Remote-Vorrichtung (300, 400) verknüpft sind, die einen Remote-Wert eines Personalisierungsparameters angibt, betriebsfähig ist;
wobei der Schlüssel (130) für die sichere Übertragung ferner zum Ermöglichen des Benutzers angepasst ist, um den Remote-Wert des Personalisierungsparameters nur dann an den Druckregler (110) zu übertragen, falls das kontaktlos lesbare Symbol (302) der Remote-Vorrichtung authentifiziert wurde und nur dann, wenn der Druckregler (110) nicht betriebsfähig ist; und
wobei der Druckregler (110) ferner auf den übertragenen Remote-Wert zum sicheren Regeln des Drucks in der angeschlossenen Tourniquet-Manschette während des Druckregelungszeitraums reagiert.

10. Tourniquet-System nach einem der vorstehenden Ansprüche, wobei, falls das maschinenlesbare Instrumentensymbol (202) nicht authentifiziert ist, das Tourniquet-Instrument konfiguriert ist, um einen zuvor bestimmten gespeicherten Wert für mindestens einen Personalisierungsparameter zu verwenden.

11. Tourniquet-System nach einem der vorstehenden Ansprüche, wobei der Schlüssel (130) für die sichere Übertragung ferner den Betrieb des Druckreglers (110) nach der selektiven Übertragung des dargestellten Werts des Personalisierungsparameters einleitet.

## Revendications

1. Système de garrot ayant une interface optique de garrot (120) pour une personnalisation sécurisée, comprenant :
un ensemble de brassard de garrot (200) comportant :
un brassard de garrot (2, 206) ayant une poche gonflable adaptée pour une connexion à un instrument de garrot,
un symbole d'instrument lisible sans contact (202) identifiant un type d'instrument de garrot parmi une pluralité de types d'instruments de garrot, et
des données de personnalisation lisibles sans contact (204) représentant une valeur d'un paramètre de personnalisation pour l'activation sécurisée du brassard de garrot (2, 206) lorsqu'il est connecté au type identifié d'instrument de garrot ; et
un instrument de garrot comportant :
une interface optique de garrot (120) communiquant avec un régulateur de pression (110) et pouvant être activée pour la lecture et l'authentification sans contact du symbole d'instrument lisible sans contact (202) associé au brassard de garrot (2, 206) s'il s'aligne sur les données d'authentification d'instrument stockées, et pouvant être activée en outre pour la lecture sans contact des données de personnalisation lisibles sans contact (204) associées au brassard (2, 206),
dans lequel l'interface optique de garrot (120) est adaptée pour présenter sous une forme perceptible à un utilisateur la valeur du paramètre de personnalisation si le symbole de l'instrument lisible sans contact (202) a été authentifié ;
une touche de transfert sécurisée (130) qui peut être activée par l'utilisateur pour transférer manuellement et sélectivement la valeur présentée du paramètre de personnalisation au régulateur de pression (110) uniquement si le régulateur de pression (110) ne peut pas être activé ; et
le régulateur de pression (110) pouvant être connecté de manière amovible au brassard de garrot (2, 206) et réagissant à la valeur transférée du paramètre de personnalisation, dans lequel le régulateur de pression (110) peut être activé pour la régulation automatique d'un niveau de pression dans le brassard de garrot connecté (2, 206) pendant un laps de temps de régulation de pression.

2. Système de garrot selon la revendication 1 dans lequel le brassard de garrot (2, 206) est stérile, le système comprenant en outre :
un connecteur stérile (210) adapté pour connecter de manière amovible le brassard de garrot stérile (2, 206) au type identifié d'instrument de garrot ; et
dans lequel le symbole d'instrument (202) et les données de personnalisation (204) sont portés au niveau d'un emplacement permettant la lecture sans contact du symbole d'instrument (202) et des données de personnalisation (204) uniquement si l'ensemble de brassard stérile (200) est positionné par l'utilisateur au sein d'une plage prédéterminée de distances et au sein d'une plage prédéterminée d'orientations par rapport à l'interface optique de garrot (120).

3. Système de garrot selon la revendication 1 ou 2 dans lequel l'interface optique de garrot (120) comporte une unité d'affichage (122) et dans lequel l'interface optique de garrot (120) est adaptée pour présenter la valeur du paramètre de personnalisation à l'utilisateur en affichant la valeur sur l'unité d'affichage (122).

4. Système de garrot selon une quelconque revendication précédente comportant en outre une touche de rejet (132) qui peut être activée par l'utilisateur pour rejeter manuellement et sélectivement le transfert de la valeur présentée du paramètre de personnalisation.

5. Système de garrot selon la revendication 2 dans lequel la valeur du paramètre de personnalisation indique que le brassard de garrot (2, 206) est adapté à une mesure automatique d'une pression d'occlusion de membre d'un patient par l'instrument de garrot identifié.

6. Système de garrot selon la revendication 2 ou 5 dans lequel la valeur du paramètre de personnalisation indique que le brassard de garrot (2, 206) est adapté à un patient pédiatrique.

7. Système de garrot selon la revendication 2, 5 ou 6, dans lequel la valeur du paramètre de personnalisation indique que le brassard de garrot (2, 206) est adapté à une anesthésie régionale intraveineuse d'un membre d'un patient.

8. Système de garrot selon une quelconque revendication précédente dans lequel l'interface optique de garrot (120) peut en outre être activée lors de l'actionnement d'une touche de résumé clinique par l'utilisateur après le laps de temps de régulation de pression pour la génération et l'affichage de données cliniques lisibles sans contact indiquant au moins un niveau de pression correspondant à un temps au sein du laps de temps de régulation de pression, et de préférence dans lequel les données cliniques lisibles sans contact indiquent en outre la valeur présentée transférée du paramètre de personnalisation.

9. Système de garrot selon une quelconque revendication précédente comportant en outre un dispositif distant (300, 400) :
dans lequel l'interface optique de garrot (120) peut en outre être activée pour la lecture et l'authentification sans contact d'un symbole de dispositif distant lisible sans contact (302) associé au dispositif distant (300, 400) s'il s'aligne sur les données d'authentification stockées correspondant au dispositif distant (300, 400) ; et
dans lequel l'interface optique de garrot (120) peut en complément être activée pour la lecture sans contact de données lisibles sans contact associées au dispositif distant (300, 400) qui indiquent une valeur distante d'un paramètre de personnalisation ;
dans lequel la touche de transfert sécurisée (130) est en outre adaptée pour permettre à l'utilisateur de transférer en toute sécurité la valeur distante du paramètre de personnalisation au régulateur de pression (110) uniquement si le symbole de dispositif distant lisible sans contact (302) a été authentifié et uniquement lorsque le régulateur de pression (110) ne peut pas être activé ; et
dans lequel le régulateur de pression (110) réagit en outre à la valeur distante transférée pour réguler en toute sécurité la pression dans le brassard de garrot connecté pendant le laps de temps de régulation de pression.

10. Système de garrot selon une quelconque revendication précédente, dans lequel si le symbole d'instrument lisible par machine (202) n'est pas authentifié, l'instrument de garrot est conçu pour utiliser une valeur stockée prédéterminée pour au moins un paramètre de personnalisation.

11. Système de garrot selon une quelconque revendication précédente dans lequel la touche de transfert sécurisée (130) déclenche en outre une activation du régulateur de pression (110) lors du transfert sélectif de la valeur présentée du paramètre de personnalisation.
